# NEUE EUROPÄISCHE PATENTSCHRIFT

(11) **EP 1 168 980 B2**
(45) Veröffentlichungstag und Bekanntmachung des Hinweises auf die Entscheidung über den Einspruch: **14.09.2011**
(45) Hinweis auf die Patenterteilung: 13.06.2007
(21) Anmeldenummer: 00917065.5
(22) Anmeldetag: 10.04.2000
(51) Int. Cl.: A61C 9/00, A61K 6/10

(54) **ADHESIVSYSTEM FÜR SILICONE**
ADHESIVE SYSTEM FOR SILICONES
SYSTEME ADHESIF POUR SILICONE

(30) Priorität: 09.04.1999 DE 19916131
(43) Veröffentlichungstag der Anmeldung: 09.01.2002
(73) Patentinhaber: S & C Polymer Silicon- und Composite-Spezialitäten GmbH, 25335 Elmshorn (DE)
(72) Erfinder: ENGELBRECHT, Jürgen, D-25335 Elmshorn (DE)
(74) Vertreter: Forstmeyer, Dietmar
(86) Internationale Anmeldenummer: PCT/EP2000/003178
(87) Internationale Veröffentlichungsnummer: WO 2000/061028

(56) Entgegenhaltungen:
- EP-A2- 0 632 060
- EP-A2- 0 826 359
- EP-A2- 0 994 172
- WO-A-00/59453
- WO-A1-00/47165
- DE-A- 4 414 837
- DE-A- 19 635 696
- US-A- 4 763 791
- A. Boeckler, Dissertation "Dibenzoylperoxid als potentielles Allergen in Prothesenkunststoffen", 2003, S.4

## Beschreibung

Die vorliegende Erfindung betrifft ein Besteck (Kit of Parts), umfassend:
a) zumindest ein wiederanlösbares (Co-)Polymer,
b) zumindest ein Adhesiv für Silicone, gegebenenfalls
c) einen Grundkörper, und gegebenenfalls
d) eine Siliconmasse.

Abformungen mit Abformmassen insbesondere im Dentalbereich machen häufig die Verwendung sogenannter individuell geformter Abformlöffel nötig. Individuelle Abformlöffel sind individuell hergestellte Formteile in Form eines Abformlöffels, die z.B. aus selbsthärtenden Mischungen aus Methylmethacrylat/Polymethylmethacrylat oder aus lichthärtbaren Platten aus Composite (die aus mit Glaspulver versetzten mehrfunktionellen lichthärtbaren (Meth-)Acrylat-Mischungen bestehen), in dentalen Anwendungen "lichthärtbare individuelle Abformlöffelplatten" genannt, hergestellt werden.

US 4,763,791 beschreibt ein dentales Besteck, das verschiedene Mittel zur Abdrucknahme umfasst. DE 44 14 837 beschreibt eine Haftvermittlungsschicht für Zahnprothesenunterfütterungen zur dauerhaften Verbindung eines Prothesenkunststoffs mit einem Silikonunterfütterungsmaterial. Die Haftvermittlerschicht besteht aus einem Reaktionsgemisch aus einem 2-Komponenten-Polyurethan Reaktionsklebstoff, der durch eine Polyaddition von Polyolen mit di- oder mehrfunktionellen Isocyanaten gebildet wird und einem Organohydrogen-Silikonöl. DE 196 35 696 beschreibt ein haftfestes und kraftschlüssiges Adhesiv zur Haftvermittlung zwischen elastischen und starren Materialien und deren Verwendung als Haftvermittler zur dauerhaften Verbindung zwischen Prothesenkunststoff und Silikonunterfütterungsmaterial.

Üblicherweise werden im Dentalbereich Silicone als Abformmassen verwendet. Der Verbund zwischen Abformlöffeln und Siliconen stellt jedoch ein Problem dar: Silicone haften nicht von selbst auf Kunststoffen oder Compositen. Um dennoch eine Haftung zwischen Siliconabformmassen und Abformlöffeln zu gewährleisten, wurde zum einen versucht, die Abformmassen mit Hilfe von mechanischen Retentionen wie Löchern, Unterschnitten, umgebörtelten Rändern usw., also rein mechanisch, stabil im Abformlöffel zu verankern und ein Abheben aus diesem nach der Abdrucknahme zu verhindern.

In ähnlicher Weise wird in der DE 42 28 538 versucht, die Haftung zwischen einem mehrfach zu verwendenden Abformlöffel und einer Siliconabformmasse dadurch zur verbessern, daß auf den metallischen Abformlöffel eine Dispersion aufgestrichen wird und diese Dispersion getrocknet wird. Doch auch derartige Systeme weisen keine ausreichende Haftung der Siliconmassen auf den Abformlöffeln auf.

Zum anderen wurde versucht, die Haftung der Siliconmassen auf Abformlöffeln mit Hilfe von Lösungen zähklebriger Siliconpolymerer molekular-mechanisch zu verbessern. In der Regel sind diese Silicon-Abformlöffeladhesive Lösungen nur teilweise vernetzter Silicone in flüchtigen Lösungsmitteln. Da keine direkte chemische Verbindung zwischen Siliconabformmasse und den teilvernetzten Siliconpolymeren des Abformlöffeladhesivs stattfindet, können diese Adhesive sowohl für kondensationsvernetzende als auch für additionsvernetzende Siliconabformmassen verwendet werden.

Beide vorstehend erwähnten Maßnahmen zur besseren Haftung der Siliconabformmassen auf den Abformlöffeln stellen jedoch unzureichende Lösungen dar: Besonders schwierig ist das Einbringen von mechanischen Retentionen in individuell gefertigte Abformlöffel, da diese z.B. nachträglich durch Bohren hergestellt werden müssen. Bei diesen harten Löffeln führt Bohren außerdem leicht zu Spannungsrissen; zu viele mechanische Lochretentionen schwächen zudem die Bruchfestigkeit und Verbiegefestigkeit der Abformlöffel.

Die auf dem Markt befindlichen lösungsmittelhaltigen zähklebrigen Adhesive zeigen nur eine unterstützende Haftwirkung: Bei etwas stärkeren zugkräften beim Entfernen des Abdrucks vom abzuformenden Objekt wie einem Zahn hebt sich der Siliconabdruck unter Verformung von dem Abformlöffel ab und das Zähadhesiv löst sich unter Bildung vieler Ziehfäden. Auf Grund der Verformung des Abdrucks kommt es leicht zu Fehlpassungen.

Es ist vor kurzem gefunden worden, daß Adhesive, die genutzt werden, um weichbleibende Siliconunterfütterungsmaterialien an Prothesenkunststoff fest zu binden (z.B. beigefügte Patentanmeldung DE 199 05 224.7, Patentanmeldung DE 196 35 696 A1 oder Patent EP 0 632 063 A1), auch hervorragend auf Abformlöffeln haften, die leicht anlösbar sind. Anlösbare Löffel sind z.B. solche aus Polystyrol, Polycarbonat. Auf individuellen Abformlöffeln auf Monomer/Polymer-Basis wie z.B. aus Methylmethacrylat/Polymethylmethacrylat können diese Adhesive zu einer guten Haftung der Siliconabformmasse auf eben diesem Typ von individuellen Abformlöffeln führen.

Problematisch ist jedoch das Erzeugen einer guten Haftfestigkeit von Siliconen auf der Oberfläche von vernetzten Methacrylaten, speziell auf individuellen lichthärtbaren Abformlöffeln auf Methacrylat-Composite-Basis, die in großer Zahl verwendet werden. Auf diesen Typen von polymerisierten Besteckn versagen die genannten neuen Haftprimer für Silicone.

Aufgabe der Erfindung war es daher, ein Besteck bereitzustellen, das einen leicht herzustellenden und sicheren Verbund zwischen Abformlöffeln wie aus Methacrylat-Compositen bestehenden Abformlöffeln und insbesondere speziellen lichthärtbaren individuellen Abformlöffeln und Siliconmassen ermöglicht, wobei der Verbund in etwa so stark ist wie die Kohäsion der Siliconmasse selbst.

Erfindungsgemäß wird die Aufgabe gelöst durch ein Besteck nach Anspruch 1, das
a) zumindest ein wiederanlösbares (Co-)Polymer,
b) zumindest ein Adhesiv für Silicone, gegebenenfalls
c) einen Grundkörper, und gegebenenfalls
d) eine Siliconmasse umfaßt.

Derartige Bestecke weisen den Vorteil auf, daß es zu einer chemischen Verbindung zwischen dem Adhesiv und der Siliconmasse kommen kann. Dadurch wird die Haftung der Siliconmasse - über das Adhesiv und das wiederanlösbare (Co-)Polymer - auf dem Grundkörper extrem fest. Eine derartige feste Haftung kann über bekannte Systeme, insbesondere Systeme, die hauptsächlich auf mechanischer Haftung beruhen, nicht erreicht werden.

Erfindungsgemäß kann der Grundkörper z.B. Polymere oder Copolymere von Methacrylaten umfassen oder daraus bestehen. Er kann ein Compositkörper sein und zusätzlich Füllstoffe wie Glaspulver umfassen.

Bei dem gegebenenfalls umfaßten Grundkörper kann es sich um einen Abformlöffel, insbesondere um einen individuell hergestellten Abformlöffel und besonders bevorzugt um einen individuell hergestellten lichthärtenden Abformlöffel handeln. Auch kann es sich bei dem Grundkörper um dentale Kronen oder Brücken oder provisorische Kronen oder Brücken, oder um Bißschienen aus Compositematerial handeln.

Die löslichen bzw. (wieder-)anlösbaren (Co-)Polymere können z.B. Polystyrol, Polycarbonat, Poly(meth-)acrylat, Polyvinyl-chlorid, Polysulfon, Polymethylpenten oder Polystyrolacrylnitril oder Gemische davon umfassen oder sein, wobei die Poly(meth-)acrylate wegen deren chemischer Verwandtschaft mit den vernetzten Methacrylaten vorzugsweise Polymere oder Mischpolymerisate von Methyl-, Ethyl-, Propyl-, Butyl-, Neopentyl- oder Tetrahydrofurfurylestern von Acrylsäuren oder Methacrylsäuren sind.

Prinzipiell ist jedoch jedes lösliche bzw. (wieder-)anlösbare Polymere geeignet, welches sich in ausreichender Stärke mit (co-) polymerisierten Grundkörpern insbesondere auf Basis von mit Füllkörpern versetzten oder keine Füllkörper enthaltenden Dimethacrylaten oder anderen mehrfunktionellen Methacrylaten verbinden kann. Eine Verbindung ist ausreichend stark, wenn sie eine Haftkraft von zumindest 50%, stärker bevorzugt von zumindest 90% und am stärksten bevorzugt von zumindest 100% der Kohäsionskraft der eingesetzten Abformmasse aufweist.

Erfindungsgemäß werden vorzugsweise Adhesive für Siliconmassen eingesetzt, die mit der Siliconmasse chemisch reagieren, d.h. chemische Bindungen mit der Siliconmasse aufbauen können.

Derartige Adhesive für Silicone können z.B. ein (Co-) Polymeres oder ein Gemisch von (Co-) Polymeren sein, das/die SiH- und/oder Vinylgruppen enthält/enthalten; insbesondere wird bevorzugt, daß das (Co-) Polymere oder das Gemisch von (Co-) Polymeren pro Einheit, die zwei ursprünglich eingesetzten Monomeren entspricht, mindestens eine SiH- oder Vinylgruppe enthält.

Derartige Adhesive sind z.B. in der beigefügten Patentanmeldung DE 199 05 224.7 offenbart, auf die auch bezüglich dieser Offenbarung ausdrücklich Bezug genommen wird. In alternativer Weise kann das Adhesiv für Silicone ein (Co-)Polymeres oder ein Gemisch von (Co-) Polymeren sein, das Silanolgruppen -Si(X)-enthält, wobei X = OH, -O-R_{c} oder Reste der Formeln 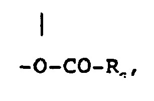 -NR_{d}-R_{c}, -NR_{d}-CO-R_{c} sind, und die Reste R_{c} und R_{d} oder die freien Bindungen am Si-Atom unabhängig voneinander H-Atome, OH-Gruppen, Alkylgruppen, Alkoxygruppen oder Arylgruppen sein bzw. aufweisen können. Es wird besonders bevorzugt, wenn die Reste der Formeln -O-R_{c}, -O-CO-R_{c}, -NR_{d}-R_{c}, -NR_{d}-CO-R_{c} leicht hydrolisierbare Reste sind. Derartige Gruppen der Formel X sind auch in der beigefügten PCT-Patentanmeldung "Haftvermittler für Siliconmaterialien", Anwaltsakte 10730, beim Europäischen Patentamt eingereicht am 06.04.2000 und der beigefügten deutschen Patentanmeldung DE 199 15 492.9 offenbart, auf die auch bezüglich dieser Offenbarung ausdrücklich Bezug genommen wird.

Die wiederanlösbaren (Co-)Polymere sind in mindestens einem leichtflüchtigen, inerten Lösungsmittel gelöst. Als Lösungsmittel kann ein aliphatischer oder aromatischer, halogenierter oder nicht-halogenierter Kohlenwasserstoff, Ether, Keton, Ester oder cyclisches Siloxan eingesetzt werden. Derartige Lösungsmittel sind dem Fachmann an sich bekannt.

Gemäß einer bevorzugten Ausführungsform sind die Adhesive für Silicone in mindestens einem leichtflüchtigen, inerten Lösungsmittel gelöst oder angelöst. Als Lösungsmittel kann ein aliphatischer oder aromatischer, halogenierter oder nicht-halogenierter Kohlenwasserstoff, Ether, Keton, Ester oder cyclisches Siloxan eingesetzt werden. Derartige Lösungsmittel sind dem Fachmann an sich bekannt.

Bei der Siliconmasse d) kann es sich z.B. um eine Siliconabformmasse zur Abformung von Zähnen handeln.

In dem erfindungsgemäßen Besteck liegen die Komponenten a), b), gegebenenfalls c) und gegebenenfalls d) getrennt, z.B. in getrennten Behältern wie Flaschen, Tuben oder Dosen etc. vor. Auch kann der Grundkörper auf andere Weise separat verpackt vorliegen.

Durch derartige Adhesivsysteme für Siliconmassen insbesondere auf Compositeformteilen wird eine innige Verbindung zwischen Siliconmasse und Compositeformteilen hergestellt.

weiter wird erfindungsgemäß ein Verfahren nach Anspruch 21 zur Herstellung eines Formteils, wie eines Abformlöffels, bereitgestellt, wobei
a) eine Lösung mindestens eines wiederanlösbaren (Co-)Polymeren auf mindestens eine Oberfläche eines Grundkörpers aufgebracht wird,
b) das/die wiederanlösbare(n) (Co-)Polymer(e) getrocknet wird/werden,
c) eine Lösung mindestens eines Adhesivs für Silicone auf die Schicht des/der wiederanlösbaren (Co-)Polymers/Polymere aufgebracht wird,
d) das/die Adhesiv(e) für Silicone getrocknet wird/ werden, und gegebenenfalls
e) eine Siliconmasse auf das/die Adhesiv(e) aufgebracht wird.

Dieses Verfahren kann z.B. von einem Zahnarzt, einem Zahntechniker oder einer zahnarzthelferin durchgeführt werden.

Ferner wird erfindungsgemäß ein Abformlöffel nach Anspruch 22 bereitgestellt, der nach dem vorstehend beschriebenen Verfahren herstellbar ist.

Die in Schritt c) eingesetzten Adhesive für Silicone sind gemäß einer bevorzugten Ausführungsform in mindestens einem der vorstehend erwähnten leichtflüchtigen, inerten Lösungsmittel gelöst.

Ein erfindungsgemäßer Abformlöffel mit einer verbesserten Haftung von Siliconmassen auf Compositeformteilen, speziell ein entsprechender individueller lichtgehärteter Abformlöffel, kann insbesondere dadurch hergestellt werden, daß
1. Lösungen von wiederanlösbaren Polymeren auf die Compositeoberfläche aufgetragen und getrocknet werden, so daß sie auf dieser Compositeoberfläche haften, und
2. Adhesivlösungen für härtbare Silicone auf die Schicht der wiederanlösbaren Polymere aufgebracht und getrocknet werden, so daß sie auf den wiederanlösbaren Polymeroberflächen haften; dadurch wird eine innige Verbindung zwischen einer aufzubringenden Siliconmasse und der anlösbaren Polymeroberfläche ermöglicht.

Beispielsweise können erfindungsgemäß individuelle Abformlöffel eingesetzt werden. Individuelle Abformlöffel, die auch mit Füllstoffen versetzte Dimethacrylate umfassen können, liegen in der Regel zunächst als vorgefertigte lichthärtbare Platten vor und werden auf einem Gipsmodel zu einem individuellen Abformlöffel modelliert und lichtgehärtet. Nach Entfernen der sauerstoffinhibierenden Schmierschicht mit Ethanol wird erfindungsgemäß z.B. ein dünner Film eines anlösbaren Polymeren auf die Oberfläche des individuellen Abformlöffels aufgetragen. Dieses geschieht leicht durch Auftrag des in leichtflüchtigen inerten Lösungsmitteln gelösten Polymeren.

Wie vorstehend beschrieben, werden mit anlösbaren Polymeren behandelte Grundkörper wie Nethacrylatgrundkörper getrocknet und dann erfindungsgemäß in einem zweiten Schritt mit Siliconhaftvermittlern (Primern) behandelt, wie sie z.B. in der beigefügten Patentanmeldung DE 199 05 224.7 oder in der Patentanmeldung DE 196 35 696 A1 oder im Patent EP 0 632 060 A1 für additionsvernetzende Systeme (A) beschrieben sind, oder auch wie sie z.B. in der beigefügten PCT-Patentanmeldung "Haftvermittler für Siliconmaterialien", Anwaltsakte 10730, beim Europäischen Patentamt eingereicht am 06.04.2000 und der beigefügten deutschen Patentanmeldung DE 199 15 492.9, für kondensationsvernetzende Systeme (K) beschrieben sind.

Geeignete Primer (A) für die Beschichtung von Grundkörpern, speziell von individuellen lichtgehärteten Abformlöffeln, mit additionsvernetzenden Abformmassen können Lösungen von Haftpolymeren sein, die eine ausreichende Menge an SiH- und/oder Vinyl-Gruppen enthalten und die sich ausreichend in der aufgetragenen Polymeroberfläche des Dimethacrylatgrundkörpers lösen können, wobei Lösungsmittel verwendet werden, die ausreichend flüchtig sind. Insbesondere können die vorstehend beschriebenen Lösungsmittel eingesetzt werden. So behandelte Abformlöffel sind für eine starke Haftung additionsvernetzender Silicone vorbereitet und geeignet.

Geeignete Primer (K) für die Beschichtung von Abformlöffeln mit kondensationsvernetzenden Abformmassen können z. B. Lösungen von Polymeren sein, die eine ausreichende Menge an Silanolgruppen - Si(X)- enthalten, wobei X = OH, -O-R_{c}, -O-CO-R_{c}, 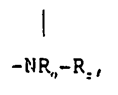 -NR_{d}-CO-R_{c} und die Reste R_{c} und R_{d} wie vorstehend def i-niert sind, wobei sich die Lösungen der Polymere ausreichend in der aufgetragenen Polymeroberfläche (1. Schicht) des Dimethacrylat-Abformlöffels lösen können und die verwendeten Lösungsmittel ausreichend flüchtig sind. Insbesondere können die vorstehend beschriebenen Lösungsmittel eingesetzt werden. So behandelte Abformlöffel sind für eine starke Haftung kondensationsvernetzender Silicone auf dieser Fläche vorbereitet und geeignet.

Die erfindungsgemäßen Bestecke und Abformlöffel können für medizinische Anwendungen, insbesondere für dentale Anwendungen zum Einsatz kommen, wobei ihre Verwendung als Abformlöffel für Siliconmassen besonders bevorzugt wird. Bei den eingesetzten Abformmassen handelt es sich z.B. um Siliconmassen zur Abformung von Zähnen.

Im Falle der individuellen Abformlöffel können die so vorbereiteten Löffel beim Zahnarzt direkt mit Abformmasse des entsprechenden Härtungstyps beschickt und in den Mund zum Zwecke der Abformung verbracht werden. Bei der Herausnahme des beschickten Abformlöffels bleiben Abformmasse und Abformlöffel fest verbunden. Ein Ablösen von Teilen der ausgehärteten Abformmasse vom Abformlöffel geschieht nicht, es treten keine entsprechenden Distorsionen und Fehlabformungen auf. Versuche, die Abformmasse vom Abformlöffel zu entfernen, führen zu Abrissen von Abformmaterial.

Viele andere Anwendungen von Verbindungen von Siliconen auf vernetzten Methacrylatkunststoffen sind vorstellbar und erfindungsgemäß.

So können beispielsweise mit Hilfe des erfindungsgemäßen Adhesivsystems am unteren Saum von mit Composit verblendeten dentalen Kronen oder Brücken oder provisorischen Kronen oder Brücken auf Basis von Dimethacrylaten, elastische Zahnfleischmasken aus Silicon zur Abdeckung ungenügender Zahnfleischsäume befestigt werden.

So können beispielsweise mit Hilfe der erfindungsgemäßen Adhesivsysteme die Innenseite von Composite-Aufbißschienen behandelt und mit härtendem Silicon elastisch unterfüttert werden.

weiter können bei Kontaktallergien von Patienten gegen Inhaltstoffe von Compositen diese nach patentgemäßer Vorgehensweise mit einer dünnen Schicht härtenden Silicons überzogen werden, oder Compositeaformkörper bei industriellen Anwendungen in patentgemäßer Weise beschichtet und mit einem Siliconkleber dauerhaft elastisch verbunden werden.

### BEISPIELE

Haftung von additionsvernetzender Siliconabformmasse auf einem lichthärtbaren individuellen Abformlöffel.

Eine lichthärtbare Abformlöffelplatte (Megatray, Fa. Megadenta, Deutschland) wird auf einem Gipsmodel zu einem Abformlöffel geformt und in einer Bestrahlungseinheit (Megaflash, Fa. Megadenta) 3 min lichtgehärtet. Die sauerstoffinhibierende Schicht auf der Oberfläche wird mit einem alkoholgetränkten Tuch auf der Innenfläche des so erhaltenen Abformlöffels entfernt.

Es wird eine 15%ige Lösung eines löslichen Polymerpulvers (Polymethylmethacrylat MW332, Fa. Röhm, Deutschland) in Toluol hergestellt und diese auf die gesäuberte Innenfläche des Abformlöffels dünn aufgetragen und trocknen gelassen.

Auf diese so behandelte Innenfläche wird nun der Siliconhaftprimer entsprechend dem Beispiel 2 der beigefügten Patentanmeldung DE 199 05 224.7 aufgetragen und getrocknet. Der Abformlöffel wird nun mit der additionsvernetzenden Siliconvorabformmasse R-si-line Putty (R-Dental, Deutschland) beladen und eine Abformung eines bezahnten Oberkiefers im Munde vorgenommen. Nach Aushärtung wird der Abformlöffel mit der ausgehärteten Abformmasse ohne eine Spur von Ablösung des Silicons von der Abformlöffelinnenfläche entnommen. Die Abformmasse ist fest mit dem Abformlöffel verbunden kann nur unter Zerstörung der Abformmasse entfernt werden.

## Patentansprüche

1. Besteck für dentale Anwendungen, umfassend:
a) zumindest ein Polymer oder Copolymer, und
b) zumindest ein Adhesiv für Silicone,
**dadurch gekennzeichnet, dass** das zumindest eine Polymer oder Copolymer wiederanlösbar ist, und
das zumindest eine wiederanlösbare Polymer oder Copolymer in mindestens einem leichtflüchtigen, inerten Lösungsmittel gelöst ist, und
die Komponenten a) und b) in getrennten Behältern vorliegen.

2. Besteck nach Anspruch 1, **dadurch gekennzeichnet, daß** es zusätzlich
c) mindestens einen Grundkörper umfaßt.

3. Besteck nach Anspruch 1 oder 2, **dadurch gekennzeichnet, daß** es zusätzlich
d) mindestens eine Siliconmasse umfaßt.

4. Besteck nach einem der vorstehenden Ansprüche 2-3, **dadurch gekennzeichnet, daß** der Grundkörper Polymere oder Copolymere von Methacrylaten umfaßt oder aus diesen besteht.

5. Besteck nach einem der vorstehenden Ansprüche 2-4, **dadurch gekennzeichnet, daß** der Grundkörper zusätzlich Füllstoffe wie Glaspulver umfaßt.

6. Besteck nach einem der vorstehenden Ansprüche 2-5, **dadurch gekennzeichnet, daß** es sich bei dem Grundkörper um einen Abformlöffel handelt.

7. Besteck nach einem der vorstehenden Ansprüche 2-6, **dadurch gekennzeichnet, daß** es sich bei dem Grundkörper um einen individuell hergestellten Abformlöffel handelt.

8. Besteck nach einem der vorstehenden Anspruche 2-7, **dadurch g**ekennzeichnet, daß es sich bei dem Grundkörper um einen individuell hergestellten lichthärtenden Abformlöffel handelt.

9. Besteck nach einem der Ansprüche 2 bis 5, **dadurch gekennzeichnet, daß** es sich bei dem Grundkörper um dentale Kronen oder Brücken oder provisorische Kronen oder Brücken handelt.

10. Besteck nach einem der vorstehenden Ansprüche, **dadurch gekennzeichnet, daß** die wiederanlösbaren Polymere oder Copolymere Polystyrol, Polycarbonat, Poly(meth-)acrylat, Polyvinylchlorid, Polysulfon, Polymethylpenten oder Polystyrolacrylnitril oder Gemische davon umfassen oder sind.

11. Besteck nach Anspruch 10, **dadurch gekennzeichnet, daß** die Poly(meth-)acrylate Polymere oder Mischpolymerisate von Methyl-, Ethyl-, Propyl-, Butyl-, Neo-pentyl- oder Tetrahydrofurfurylestern von Acrylsäuren oder Methacrylsäuren sind.

12. Besteck nach einem der vorstehenden Ansprüche, **dadurch gekennzeichnet, daß** das Adhesiv für Silicone ein Polymer oder Copolymer oder ein Gemisch von Polymeren und/oder Copolymeren ist, das/die SiH- oder vinylgruppen enthält/enthalten.

13. Besteck nach Anspruch 12, **dadurch gekennzeichnet, daß** das Polymere oder Copolymere oder das Gemisch von Polymeren und/oder Copolymeren pro Einheit, die zwei ursprünglich eingesetzten Monomeren entspricht, mindestens eine SiH- oder Vinylgruppe enthält.

14. Besteck nach einem der Ansprüche 1 bis 11, **dadurch gekennzeichnet, daß** das Adhesiv für Silicone ein Polymer oder Copolymer oder ein Gemisch von Polymeren und/oder Copolymeren ist, das Silanolgruppen enthält, wobei X = OH, -O-R_{c} oder Reste der Formeln 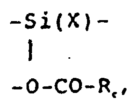 -NR_{d}-R_{c}, -NR_{d}-CO-R_{c} sind, wobei die Reste R_{c} und R_{d} unabhängig voneinander H-Atome, OH-Gruppen, Alkylgruppen, Alkoxygruppen oder Arylgruppen sein können.

15. Besteck nach Anspruch 14, **dadurch gekennzeichnet, daß** die Reste der Formeln -O-R_{c}, -O-CO-R_{c}, -NR_{d}-R_{c}, -NR_{d}-CO-R_{c} leicht hydrolisierbare Reste sind.

16. Besteck nach einem der vorstehenden Ansprüche, **dadurch gekennzeichnet, daß** die Adhesive für Silicone in mindestens einem leichtflüchtigen, inerten Lösungsmittel gelöst oder angelöst sind.

17. Besteck nach Anspruch 16, **dadurch gekennzeichnet, daß** das Lösungsmittel ein aliphatischer oder aromatischer, halogenierter oder nicht-halogenierter Kohlenwasserstoff, Ether, Keton, Ester oder cyclisches Siloxan ist.

18. Besteck nach einem der vorstehenden Ansprüche, **dadurch gekennzeichnet, daß** die Siliconmasse d) eine Siliconabformmasse zur Abformung von Zähnen ist.

19. Besteck nach einem der vorstehenden Ansprüche, **dadurch gekennzeichnet, daß** die Komponenten a), b), gegebenenfalls c) und gegebenenfalls d) getrennt vorliegen.

20. Besteck nach einem der vorstehenden Ansprüche, **dadurch gekennzeichnet, daß** die Komponenten a), b), gegebenenfalls c) und gegebenenfalls d) in getrennten Flaschen oder Dosen vorliegen.

21. Verfahren zur Herstellung eines Formteils unter Verwendung eines Bestecks nach einem der vorstehenden Ansprüche, **dadurch gekennzeichnet, daß**
a) eine Lösung mindestens eines wiederanlösbaren Polymeren oder Copolymeren nach einem der vorstehenden Ansprüche auf mindestens eine Oberfläche eines Grundkörpers nach einem der vorstehenden Ansprüche aufgebracht wird,
b) das/die wiederanlösbare(n) Polymer(e) oder Copolymer(e) getrocknet wird/werden,
c) eine Lösung mindestens eines Adhesivs für Silicone nach einem der vorstehenden Ansprüche auf die Schicht des/der wiederanlösbaren Polymers/Polymere oder Copolymers/Copolymere aufgebracht wird,
d) das/die Adhesiv(e) für Silicone getrocknet wird/ werden, und gegebenenfalls
e) eine Siliconmasse auf das/die Adhesiv(e) aufgebracht wird.

22. Formteil für dentale Anwendungen umfassend:
a) einen Grundkörper,
b) ein auf mindestens eine Oberfläche des Grundkörpers aufgebrachtes wiederanlösbares Polymer oder Copolymer,
c) ein auf eine Schicht eines wiederanlösbaren Polymers oder Copolymers aufgebrachtes Adhesiv für Silicone, und gegebenenfalls
d) eine auf ein Adhesiv aufgebrachte Siliconmasse;
wobei die Schichten a) bis d) Materialien umfassen wie sie in einem der vorstehenden Ansprüche definiert sind.

23. Formteil nach Anspruch 22, **dadurch gekennzeichnet daß** es sich bei dem Grundkörper um einen Abformlöffel handelt.

24. Verwendung eines Bestecks nach einem der Ansprüche 1 bis 20 oder eines Formteils nach Anspruch 22 oder 23 zur Abformung für medizinische Anwendungen.

25. Verwendung nach Anspruch 24 zur Abformung für dentale Anwendungen.

26. Verwendung nach Anspruch 24, **dadurch gekennzeichnet, daß** es sich bei den Siliconmassen um Massen zur Abformung von Zähnen handelt.

## Claims

1. Kit of parts for dental applications, comprising:
a) at least one polymer or copolymer, and,
b) at least one adhesive for silicones,
**characterized in that** the at least one polymer or copolymer is resoluble, and
the at least one resoluble polymer or copolymer is dissolved in at least one readily volatile inert solvent, and
components a) and b) are present in separate containers.

2. Kit of parts according to claim 1, **characterised in that** it comprises additionally
c) at least one base body.

3. Kit of parts according to claims 1 or 2, **characterised in that** it comprises additionally
d) at least one silicone composition.

4. Kit of parts according to any one of the preceding claims 2 to 3, **characterised in that** the base body comprises or consists of polymers or copolymers of methacrylates.

5. Kit of parts according to any one of the preceding claims 2 to 4, **characterised in that** the base body additionally comprises fillers, such as glass powder.

6. Kit of parts according to any one of the preceding claims 2 to 5, **characterised in that** the base body is an impression tray.

7. Kit of parts according to any one of the preceding claims 2 to 6, **characterised in that** the base body is an individually manufactured impression tray.

8. Kit of parts according to any one of the preceding claims 2 to 7, **characterised in that** the base body is an individually manufactured photo-curing impression tray.

9. Kit of parts according to any one of claims 2 to 5, **characterised in that** the base body is a dental crown or bridge or a temporary crown or bridge.

10. Kit of parts according to any one of the preceding claims, **characterised in that** the resoluble polymers or copolymers comprise or are polystyrene, polycarbonate, poly(meth-)acrylate, polyvinyl chloride, polysulphone, polymethylpentene or polystyrene acrylonitrile or mixtures thereof.

11. Kit of parts according to claim 10, **characterised in that** the poly(meth-)acrylates are polymers or mixed polymerisates of methyl, ethyl, propyl, butyl, neopentyl or tetrahydrofurfuryl esters of acrylic acids or methacrylic acids.

12. Kit of parts according to any one of the preceding claims, **characterised in that** the adhesive for silicones is a polymer or copolymer or a mixture of polymers and/or copolymers containing SiH or vinyl groups.

13. Kit of parts according to claim 12, **characterised in that** the polymer or copolymer or the mixture of polymers and/or copolymers contains at least one SiH or vinyl group per unit, each unit corresponding to two originally used monomers.

14. Kit of parts according to any one of claims 1 to 11, **characterised in that** the adhesive for silicones is a polymer or copolymer or a mixture of polymers and/or copolymers containing silanol groups -Si(X)- wherein X = OH, -O-R_{c} or radicals of the formulae 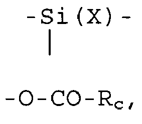 -NR_{d}-R_{c}, -NR_{d}-CO-R_{c}, wherein the radicals R_{c} and R_{d} each independently of the other may be H atoms, OH groups, alkyl groups, alkoxy groups or aryl groups.

15. Kit of parts according to claim 14, **characterised in that** the radicals of formulae -O-R_{c}, -O-CO-R_{c}, -NR_{d}-R_{c}, -NR_{d}-CO-R_{c} are readily hydrolysable radicals.

16. Kit of parts according to any one of the preceding claims, **characterised in that** the adhesives for silicones are dissolved or partially dissolved in at least one readily volatile inert solvent.

17. Kit of parts according to claim 16, **characterised in that** the solvent is an aliphatic or aromatic, halogenated or non-halogenated hydrocarbon, ether, ketone, ester or cyclic siloxane.

18. Kit of parts according to any one of the preceding claims, **characterised in that** the silicone composition d) is a silicone impression composition for taking impressions of teeth.

19. Kit of parts according to any one of the preceding claims, **characterised in that** components a), b), optionally c) and optionally d) are present separately.

20. Kit of parts according to any one of the preceding claims, **characterised in that** components a), b), optionally c) and optionally d) are present in separate bottles or cans.

21. Method of manufacturing a moulding by using a kit of parts according to any one of the preceding claims, **characterised in that**
a) a solution of at least one resoluble polymer or copolymer according to any one of the preceding claims is applied to at least one surface of a base body according to any one of the preceding claims,
b) the resoluble polymer(s) or copolymer(s) is/are dried,
c) a solution of at least one adhesive for silicones according to any one of the preceding claims is applied to the layer of the resoluble polymer(s) or copolymer(s),
d) the adhesive(s) for silicones is/are dried, and optionally
e) a silicone composition is applied to the adhesive(s).

22. Moulding for dental applications, comprising:
a) a base body,
b) a resoluble polymer or copolymer applied to at least one surface of the base body,
c) an adhesive for silicones applied to a layer of a resoluble polymer or copolymer, and optionally
e) a silicone composition applied to an adhesive.
wherein the layers a) to d) comprise materials as defined in any one of the preceeding claims.

23. Moulding according to claim 22, **characterised in that** the base body is an an impression tray.

24. Use of a kit of parts according to any one of claims 1 to 20 or of a moulding according to claims 22 or 23 for taking impressions for medical applications.

25. Use according to claim 24 for taking impressions for dental applications.

26. Use according to claim 24, **characterised in that** the silicone compositions are compositions for taking impressions of teeth.

## Revendications

1. Kit pour applications dentaires, comprenant :
a) au moins un polymère ou un copolymère, et
b) au moins un adhésif pour silicone,
**caractérisé en ce qu'**au moins un polymère ou un copolymère est résoluble, et
le au moins un polymère ou copolymère résoluble est dissous dans au moins un solvant inerte et facilement volatile, et
les composants a) et b) se présentent dans des conteneurs séparés.

2. Kit selon la revendication 1, **caractérisé en ce qu'**il comprend en outre
c) au moins un corps de base.

3. Kit selon la revendication 1 ou 2, **caractérisé en ce qu'**il comprend en outre
d) au moins une matière en silicone.

4. Kit selon l'une des revendications précédentes 2 à 3, **caractérisé en ce que** le corps de base comprend du polymère ou du copolymère de méthacrylate ou est constitué de ces derniers.

5. Kit selon l'une des revendications précédentes 2 à 4, **caractérisé en ce que** le corps de base comprend en outre des matières de remplissage comme de la poudre de verre.

6. Kit selon l'une des revendications précédentes 2 à 5, **caractérisé en ce qu'**il s'agit, pour le corps de base, d'un porte-empreinte.

7. Kit selon l'une des revendications précédentes 2 à 6, **caractérisé en ce qu'**il s'agit, pour le corps de base, d'un porte-empreinte fabriqué individuellement.

8. Kit selon l'une des revendications précédentes 2 à 7, **caractérisé en ce qu'**il s'agit, pour le corps de base, d'un porte-empreintes durcissant à la lumière fabriqué individuellement.

9. Kit selon l'une des revendications 2 à 5, **caractérisé en ce qu'**il s'agit, pour le corps de base, de couronnes ou de bridges dentaires ou de couronnes ou de bridges provisoires.

10. Kit selon l'une des revendications précédentes, **caractérisé en ce que** les polymères ou copolymères résolubles contiennent les éléments suivants ou en sont constitués : du polystyrène, du polycarbonate, du poly(méth)acrylate, du polychlorure de vinyle, du polysulfone, du polyméthylpentène ou du polystyrène-acrylonitrile ou des mélanges de ces derniers.

11. Kit selon la revendication 10, **caractérisé en ce que** les poly(méth)acrylates sont des polymères ou des copolymères d'ester méthylique, d'ester éthylique, d'ester propylique, d'ester butylique, d'ester néopentylique ou d'ester tétrahydrofurfurylique d'acides acryliques ou d'acides méthacryliques.

12. Kit selon l'une des revendications précédentes, **caractérisé en ce que** l'adhésif pour silicone est un polymère ou un copolymère ou un mélange de polymères et/ou de copolymères qui contient/contiennent des groupes de SiH ou de vinyle.

13. Kit selon la revendication 12, **caractérisé en ce que** le polymère ou le copolymère ou le mélange de polymères et/ou de copolymères par unité, correspondant à deux monomères utilisés initialement, contient au moins un groupe de SiH ou de vinyle.

14. Kit selon l'une des revendications 1 à 11, **caractérisé en ce que** l'adhésif pour silicone est un polymère ou un copolymère ou un mélange de polymères et/ou de copolymères qui contient des groupes de silanol où X = OH, -O-R_{c} ou des restes des formules sont 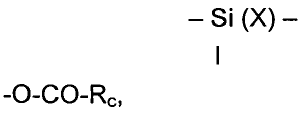 NR_{d}-R_{c}, -NR_{d}-CO-R_{c}, tandis que les restes R_{c} et R_{d} peuvent indépendamment les uns des autres être des atomes de H, des groupes OH, des groupes d'alkyle, des groupes d'alcoxy ou des groupes d'aryle.

15. Kit selon la revendication 14, **caractérisé en ce que** les restes des formules -O-R_{c}, -O-CO-R_{c}, -NR_{d}-R_{c}, -NR_{d}-CO-R_{c} sont des restes qui sont facilement hydrolisables.

16. Kit selon l'une des revendications précédentes, **caractérisé en ce que** les adhésifs pour silicone sont dissous ou dissous partiellement dans au moins un solvant inerte et facilement volatile.

17. Kit selon la revendication 16, **caractérisé en ce que** le solvant est un hydrocarbure aliphatique ou aromatique, halogéné ou non halogéné, de l'éther, de la cétone, de l'ester ou du siloxane cyclique.

18. Kit selon l'une des revendications précédentes, **caractérisé en ce que** la matière en silicone d) est une matière pour empreinte en silicone pour prendre l'empreinte de dents.

19. Kit selon l'une des revendications précédentes, **caractérisé en ce que** les composants a), b), le cas échéant c) et le cas échéant d) sont présentés séparément.

20. Kit selon l'une des revendications précédentes, **caractérisé en ce que** les composants a), b), le cas échéant c) et le cas échéant d) se présentent dans des bouteilles ou des boîtes séparés.

21. Procédé de fabrication d'une pièce moulée en utilisant un kit selon l'une des revendications précédentes, **caractérisé en ce que**
a) une solution d'au moins un polymère ou copolymère résoluble selon l'une des revendications précédentes est appliquée sur au moins une surface d'un corps de base selon l'une des revendications précédentes,
b) le/les polymère(s) ou copolymère(s) résoluble(s) est/sont séché(s)
c) une solution d'au moins un adhésif pour silicone selon l'une des revendications précédentes est appliquée sur la couche du/des polymère(s) ou copolymère(s) résoluble(s),
d) l'adhésif/les adhésifs pour silicone est/sont séché(s) et le cas échéant
e) une matière en silicone est appliquée sur l'adhésif/les adhésifs.

22. Pièce moulée pour applications dentaires comprenant :
a) un corps de base
b) un polymère ou copolymère résoluble appliqué sur au moins une surface du corps de base,
c) un adhésif pour silicone appliqué sur une couche d'un polymère ou copolymère résoluble et le cas échéant
d) une matière en silicone appliquée sur un adhésif ;
les couches a) à d) comprenant des matières telles que définies dans l'une des revendications précédentes.

23. Pièce moulée selon la revendication 22, **caractérisée en ce qu'**il s'agit, pour le corps de base, d'un porte-empreinte.

24. Utilisation d'un kit selon l'une des revendications 1 à 20 ou d'une pièce moulée selon la revendication 22 ou 23 pour faire une prise d'empreinte pour des applications médicales.

25. Utilisation selon la revendication 24 pour faire une prise d'empreinte pour des applications dentaires.

26. Utilisation selon la revendication 24, **caractérisée en ce qu'**il s'agit pour les matières en silicone de matières permettant la prise d'empreinte de dents.
